# EUROPEAN PATENT APPLICATION

(11) **EP 3 981 371 A1**
(43) Date of publication of application: **13.04.2022**
(21) Application number: 20813131.8
(22) Date of filing: 25.05.2020
(51) Int. Cl.: A61F 13/494, A61F 13/49

(54) **ABSORBENT ARTICLE**

(30) Priority: 31.05.2019 JP 2019102745
(71) Applicant: Kao Corporation, Chuo-ku Tokyo 103-8210 (JP)
(72) Inventor: SUZUKI, Takeshi, Haga-gun, Tochigi 321-3497 (JP); KOUTA, Takuya, Haga-gun, Tochigi 321-3497 (JP); TOMITA, Mina, Haga-gun, Tochigi 321-3497 (JP)
(74) Representative: Vossius & Partner Patentanwälte Rechtsanwälte mbB
(86) International application number: PCT/JP2020/020510
(87) International publication number: WO 2020/241557

(57) **Abstract**

An absorbent article (1) includes a topsheet (2), a backsheet (3) and an absorbent member (4) located between these sheets, and includes longitudinal direction (D1) corresponding to a front-rear direction of a wearer and a width direction (D2) orthogonal to the longitudinal direction (D1). The absorbent article (1) includes a composite stretch portion (10) capable of extending and contracting in one direction (X), and includes a first sheet and a second sheet (11, 12), and a plurality of elastic members (13) placed between these sheets. The elastic members (13) extend along the one direction (X), and are placed at intervals in a direction (Y) orthogonal to the one direction (X). In positions overlapping the elastic members (13) in at least one of the first sheet and the second sheet, a plurality of high-density portions (15) that do not directly join the first sheet and the second sheet (11, 12) to each other are intermittently formed in an extending direction of the elastic members (13). In the composite stretch portion (10) in a contracted state, a fold structure that is deformed so as to have ridges (17a), and grooves (18a) each including the high-density portion (15) in a bottom portion is formed.

## Description

### Technical Field

The present invention relates to an absorbent article.

### Background Art

In absorbent articles such as a disposable diaper, it has been studied to use a composite stretch sheet with a structure in which an elastic member in a stretched state is sandwiched between a plurality of sheets, for the purpose of forming folds having an excellent appearance. For example, Patent Literature 1 describes an absorbent article provided with gathers formed of a composite stretch portion. Patent Literature 1 also indicates that in the composite stretch portion, an outer sheet and an inner sheet are joined to each other by intermittent joined portions in an extension and contraction direction of the composite stretch portion and a direction orthogonal to the extension and contraction direction, so that the elastic members are placed so as not to pass through the joined portions, and both the sheets form a plurality of folds continuously extending over a plurality of elastic members.

Furthermore, Patent Literature 2 describes an elastic composite sheet formed of a first sheet and a second sheet, and a plurality of elastic members placed between the first sheet and the second sheet. The first sheet and the second sheet are joined in a plurality of joined regions. Patent Literature 2 also indicates that the joined regions are placed intermittently or continuously in an orthogonal direction to the extension and contraction direction of the elastic member, in a pattern intermittent in the extension and contraction direction of the elastic member and are placed to overlap the elastic member joined region to be fixed between the first sheet and the second sheet.

Furthermore, Patent Literature 3 describes a stretch structure of an absorbent article that includes a first sheet and a second sheet, and a plurality of elastic members provided between these first sheet and second sheet, wherein both the first sheet and the second sheet are joined by adhesion processing placed intermittently in an extension and contraction direction, and continuing in a direction to intersect the extension and contraction direction to form sheet joined portions.

### Citation List

### Patent Literature

Patent Literature 1: JP2008-132024A
Patent Literature 2: JP2009-148447A
Patent Literature 3: JP2014-198180A

### Summary of Invention

The present invention relates to an absorbent article including a liquid-permeable topsheet, a backsheet, and an absorbent member located between the topsheet and the backsheet, and including a longitudinal direction corresponding to a front-rear direction of a wearer and a width direction orthogonal to the longitudinal direction.

The absorbent article includes a composite stretch portion capable of extending and contracting in one direction.

The composite stretch portion includes a first sheet, a second sheet, and a plurality of elastic members placed between the first sheet and the second sheet.

The plurality of elastic members extend along the one direction, and are placed at intervals in a direction orthogonal to the one direction.

At least one sheet of the first sheet and the second sheet has a plurality of high-density portions that do not directly join the first sheet and the second sheet to each other.

The high-density portions overlap at least one elastic member of the plurality of elastic members, and a plurality of the high-density portions are formed intermittently in an extending direction of the one elastic member.

In the composite stretch portion in a contracted state, a fold structure in which the one sheet is deformed so as to have a plurality of grooves each having the high-density portion in a bottom portion and ridges located between the grooves is formed.

### Brief Description of Drawings

[Fig. 1] Fig. 1 is a perspective view schematically showing a stretched state of one example of a composite stretch portion included in an absorbent article of the present invention.
[Fig. 2] Fig. 2 is a sectional view along line I-I of the composite stretch portion shown in Fig. 1.
[Fig. 3] Fig. 3 is a perspective view schematically showing a natural state of the one example of the composite stretch portion of the absorbent article of the present invention.
[Fig. 4] Fig. 4 is a sectional view along line II-II of the composite stretch portion shown in Fig. 3.
[Fig. 5] Figs. 5(a) to (d) are plan views each showing an arrangement position of elastic members and high-density portions in a stretched state, in the composite stretch portion included in the absorbent article of the present invention.
[Fig. 6] Fig. 6 is a developed plan view schematically showing a skin-facing surface side (inner surface side) in a flat-out, uncontracted state, in one embodiment of the absorbent article of the present invention.
[Fig. 7] Fig. 7 is a sectional view along line V-V of the absorbent article shown in Fig. 6.
[Fig. 8] Fig. 8 is an essential part enlarged view in a stretched state of a leak-proof cuff included in the absorbent article shown in Fig. 6.

### Description of Embodiments

A composite stretch sheet used in the arts of Patent Literatures 1 to 3 each forms folds by the first sheet and the second sheet projecting away from each other with contraction of the elastic members included in the sheet. However, the folds formed by these arts tend to be formed by the sheets being randomly raised, so that shapes of the folds tend to be uneven, and there is also room for improvement in terms of appearance.

The present invention relates to an absorbent article in which a fold structure high in aligning performance and excellent in appearance can be formed.

Hereinafter, the present invention will be described with reference to the drawings based on a preferable embodiment thereof. An absorbent article of the present invention generally has a longitudinally oblong shape having a longitudinal direction corresponding to a direction extending from a front side of a wearer to a rear side via a crotch portion, and a width direction orthogonal to the longitudinal direction. The absorbent article has a crotch region placed in the crotch portion of the wearer, a front region and a rear region that extend front and rear the crotch region. The crotch region has an excretion part-facing portion disposed to face an excretion part of the wearer at a worn time of the absorbent article, and the excretion part-facing portion is normally located in a center part in the longitudinal direction of the absorbent article or a vicinity thereof.

The absorbent article generally includes a topsheet located on a skin-facing surface side of a wearer, a backsheet located on a non-skin-facing surface side, and an absorbent member located between both the sheets. As the topsheet, a sheet having liquid permeability is preferably used, and for example, a nonwoven fabric, a perforated film and the like can be preferably used. In the topsheet, a skin-facing surface side thereof may have a projecting-and-depressed shape. For example, on the skin-facing surface side of the topsheet, a plurality of ridges can be formed in a scattered spot shape. Alternatively, ridges and grooves that extend in one direction can be alternately formed on the skin-facing surface side of the topsheet. For such a purpose, it is also possible to form the topsheet by using two or more nonwoven fabrics.

As the backsheet, for example, a sparingly liquid permeable film, a spun bond/melt blown/spun bond laminate nonwoven fabric and the like can be used. A plurality of micropores may be provided in a sparingly liquid permeable film to give water vapor permeability to the film. For the purpose of making a feel of the absorbent article and the like better, a sheet with a good texture such as a nonwoven fabric may be laminated on an outer surface of the backsheet.

The absorbent member includes an absorbent core. The absorbent core is constituted of a fiber-stacked member of hydrophilic fibers of cellulose or the like such as pulp, a mixed fiber-stacked member of the hydrophilic fibers and an absorbent polymer, a sedimentary member of an absorbent polymer, a laminate structure in which an absorbent polymer is supported between two absorbent sheets, for example. In the absorbent core, at least a skin-facing surface thereof may be covered with a liquid-permeable core-wrap sheet, or an entire region of a surface including the skin-facing surface and a non-skin-facing surface may be covered with a core-wrap sheet. As the core-wrap sheet, tissue paper formed of hydrophilic fibers, an nonwoven fabric having liquid permeability and the like can be used, for example.

In addition to the topsheet, the backsheet and the absorbent member described above, leak-proof cuffs extending along the longitudinal direction may be placed at both side portions along the longitudinal direction on the skin-facing surface side in accordance with a specific use purpose of the absorbent article. The leak-proof cuff generally includes a base end section and a free end portion. The leak-proof cuff has the base end section on the skin-facing surface side of the absorbent article, and is raised from the skin-facing surface side. The leak-proof cuff is constituted of a sheet with liquid resistance or water repellency and air permeability. An elastic member formed of a thread rubber or the like may be placed in a stretched state at the free end portion of the leak-proof cuff or a vicinity thereof. The elastic member contracts in the worn state of the absorbent article, whereby the leak-proof cuff rises toward a body of the wearer, and a liquid excreted on the topsheet is effectively prevented from running on the topsheet and leaking outward in the width direction of the absorbent article.

Furthermore, according to a specific use purpose of the absorbent article, elastic members formed of a thread rubber or the like may be placed in a stretched state in at least one end portion in the longitudinal direction of the absorbent article, or at both side portions in the width direction of the absorbent article. These elastic members contract in the worn state of the absorbent article, and thereby waist gathers or leg gathers are formed.

As the absorbent article having the above structure, for example, an open-type disposable diaper, a pull-on disposable diaper, an incontinence pad, a sanitary napkin and the like may be cited, but the absorbent article is not limited to these.

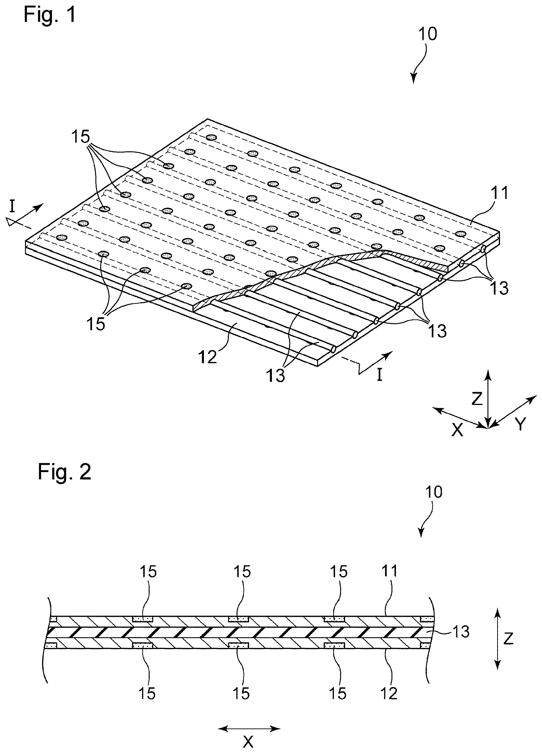

Fig. 1 and Fig. 2 show one example of a composite stretch portion constituting the absorbent article of the present invention. Fig. 1 and Fig. 2 each shows a state in which the composite stretch portion 10 is stretched. The composite stretch portion 10 constitutes a part of an absorbent article such as a disposable diaper, an incontinence pad, a sanitary napkin and the like described above.

As shown in Fig. 1 and Fig. 2, the composite stretch portion 10 has a first sheet 11, a second sheet 12, and a plurality of elastic members 13 placed between these two sheets 11 and 12. The respective elastic members 13 extend along one direction, and are intermittently placed at intervals in a direction orthogonal to the extending direction. The elastic member 13 is preferably placed throughout an entire length of the composite stretch portion 10. Thereby, the composite stretch portion 10 can extend and contract along the extending direction of the elastic member 13. In other words, the extending direction of the elastic member 13 and the extending and contracting direction of the composite stretch portion 10 correspond to each other. On the other hand, the composite stretch portion 10 does not substantially extend or contract in the direction orthogonal to the extending and contracting direction. In the following explanation, the extending direction of the elastic member 13 is also referred to as an extension and contraction direction X, and a direction orthogonal to the extension and contraction direction X is also referred to as an orthogonal direction Y.

As materials constituting the first sheet 11 and the second sheet 12, for example, sheet materials constituting the absorbent article can be used, and for example, the topsheet, the backsheet, the sheet forming the absorbent member, the sheet forming the leak-proof cuff, an outer cover forming the outer surface of the absorbent article or the like may be cited. The two sheets 11 and 12 may be formed of a same material, or may be of different materials. A mode of "a first sheet and a second sheet" in the present invention includes both of a mode of being formed of two separate sheets, and a mode of forming two surfaces facing each other by folding one continuous sheet.

The elastic member 13 causes elasticity of the composite stretch portion 10 to appear due to elasticity of the elastic member 13. The elastic member 13 may be in a shape such as a thread shape (elastic filament), a belt shape, a shape of a sheet consisting of a film or a fiber aggregate, and is preferably in a shape of a plurality of threads or belts. In the present embodiment, the respective elastic members 13 are thread-like members, and distances between the elastic members 13 adjacent in the orthogonal direction Y are substantially same. Instead of this, the distances between the elastic members 13 adjacent in the orthogonal direction Y may be different.

As shown in Fig. 1 and Fig. 2, the composite stretch portion 10 has a plurality of high-density portions 15 in a position overlapping at least one elastic member 13 in a thickness direction Z in the first sheet 11. Each of the high-density portions 15 does not directly join the two sheets 11 and 12. Likewise, each of the high-density portions 15 does not directly join the respective sheets 11 and 12 and the elastic member 13.

When focusing on one elastic member 13, a plurality of high-density portions 15 are intermittently formed along the extension and contraction direction X that is the extending direction of the elastic member 13. In the orthogonal direction Y of the composite stretch portion 10, the high-density portions 15 may be intermittently formed at intervals, or may be formed continuously. In any case, the high-density portions 15 are formed in at least one of the sheets, and preferably formed in both the sheets 11 and 12. As shown in Fig. 2, when the high-density portions 15 are formed in both the sheets 11 and 12, the high-density portion 15 formed in one of the sheets, and the high-density portion 15 formed in the other sheet are also preferably formed to overlap each other in the thickness direction Z.

The respective high-density portions 15 shown in Fig. 1 are all in a circular shape in plan view, and these are formed with intervals in both the extension and contraction direction X and the orthogonal direction Y. The shape in plan view of the high-density portion 15 may be a true circle, or may be a shape having anisotropy such as a rectangle and an ellipse. As the shape in plan view of the high-density portion 15, for example, an isotropic shape such as a true circle, a square, a regular hexagon, and a regular octagon, a polygon that is not an isotropic shape such as a triangle, an oblong, a hexagon and an octagon, and an anisotropic shape such as an ellipse, a rhombus, a heart shape, and a moon shape may be cited. When the high-density portion 15 is formed into a shape having anisotropy, the high-density portion 15 is preferably formed so that a major axis thereof extends in a direction to intersect the extension and contraction direction X, from a viewpoint of improving aligning performance and appearance of a fold structure described later. In the present description, a "major axis" refers to a long side when the shape in plan view of the high-density portion 15 is an oblong, refers to a major axis when the shape in plan view of the high-density portion 15 is an ellipse, and when the shape in plan view of the high-density portion 15 is an anisotropic shape other than an oblong and an ellipse, the "major axis" refers to a line segment in a maximum length from one side to the other.

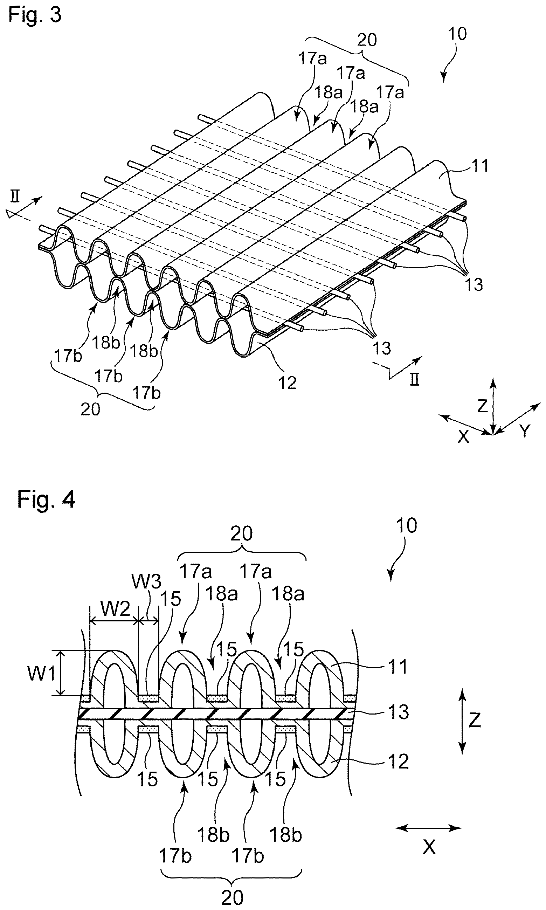

Fig. 3 and Fig. 4 show a contracted state of the composite stretch portion 10, that is, a natural state in which an external force is not applied. As shown in Fig. 3 and Fig. 4, in the first sheet 11, a plurality of first ridges 17a projecting outward away from the second sheet 12 are formed. The first ridge 17a forms a ridge shape extending in the orthogonal direction Y, and is formed in the high-density portions 15 and 15 adjacent along the extension and contraction direction X. Between the first ridges 17a adjacent along the extension and contraction direction X, a plurality of first grooves 18a each in a groove shape extending in the orthogonal direction Y are formed. The first groove 18a is formed so that the high-density portion 15 is located in a bottom portion thereof, and the high-density portion 15 in the bottom portion thereof and a vicinity of the high-density portion 15 are substantially flat. In the composite stretch portion 10, in a contracted state thereof, at least one of the sheets deforms so that a plurality of grooves each having the high-density portion in the bottom portion and a plurality of ridges are alternately formed along the extension and contraction direction X. In other words, in the composite stretch portion 10, at least one of the sheets deforms, and the fold structure 20 constituted of the pluralities of ridges 17a and grooves 18a is formed. The fold structure 20 is formed to extend in a direction to intersect the extension and contraction direction X.

In the composite stretch portion 10 shown in Fig. 3 and Fig. 4, the high-density portion 15 is also formed in the second sheet 12 in a position overlapping the high-density portion 15 formed in the first sheet 11 in the thickness direction. In the second sheet 12 shown in Fig. 3 and Fig. 4, a plurality of second ridges 17b that project outward away from the first sheet 11, and a plurality of second grooves 18b each having the high-density portion 15 in a bottom portion are alternately formed to extend in the orthogonal direction Y, similarly to the first sheet 11. In the present embodiment, the second ridge 17b of the second sheet 12 is located in a position of the first ridge 17a of the first sheet 11, and the respective ridges 17a and 17b are raised in directions opposite to each other. Furthermore, in a position of the first groove 18a of the first sheet 11, the second groove 18b of the second sheet 12 is located. Thereby, in the second sheet 12, the fold structure 20 constituted of the plurality of ridges 17b and the grooves 18b is formed. Insides of the respective ridges 17a and 17b are preferably hollow, from a viewpoint of flexibility of the fold structure.

When the high-density portion 15 is formed in a fiber sheet such as a nonwoven fabric, the high-density portion 15 is a part where constituent fibers of at least one sheet of the first sheet 11 and the second sheet 12 are densified, and a fiber density is increased as compared with other parts in the sheet. Further, in the high-density portion 15, the fiber density is high, and therefore, rigidity is increased as compared with the other parts of the sheet. When the high-density portion 15 is formed in a film, the high-density portion 15 is a part where at least one sheet of the first sheet 11 and the second sheet 12 is densified in the thickness direction, and a density is increased as compared with other parts in the sheet.

The high-density portion 15 like this can be formed by applying densification processing such as embossing to the sheet to be used in advance, or by partially applying confounding processing by machine such as needle punch and spun lace to the sheet to be used.

Furthermore, in order to arrange the elastic member 13 and the high-density portion 15 so that the elastic member 13 and the high-density portion 15 overlap each other, for example, a high-density portion column in which the high-density portions are placed in one direction is formed in the sheet, the elastic member and the sheet are caused to overlap each other so that the extending direction of the elastic member and the extending direction of the high-density portion column correspond to each other, and positioning is preferably performed so that positions of the elastic member and the high-density portion column in the thickness direction overlap each other. In a form like this, an interval between the elastic members 13 or an interval between the high-density portions 15 can be properly adjusted according to design dimensions of an intended absorbent article.

According to the absorbent article of the present invention having the above structure, the plurality of high-density portions 15 are formed along the extension and contraction direction X, so that the fold structures with the high-density portions 15 as the bottom portions are formed along the orthogonal direction Y with high aligning performance. In detail, in the high-density portion 15, rigidity is relatively high due to the fiber density being high, so that when the fold structure is formed with contraction of the elastic member, the sheet located between the adjacent high-density portions 15 easily rise outward in the thickness direction with the high-density portions 15 as start points. Meanwhile, tin the part where the high-density portion 15 is formed, unintentional rise of the sheet is suppressed. As a result, the absorbent article of the present invention has the fold structure in which the ridges and grooves that are formed are formed with regularity, aligning performance is high, and appearance is good. In addition to this, the high-density portion 15 is formed not to directly join the two sheets 11 and 12 to each other, so that there are an advantage that elasticity of the composite stretch portion 10 itself is enhanced, flexibility of the sheet constituting the composite stretch portion 10 is sufficiently maintained, and comfortability in the worn state of the absorbent article is improved.

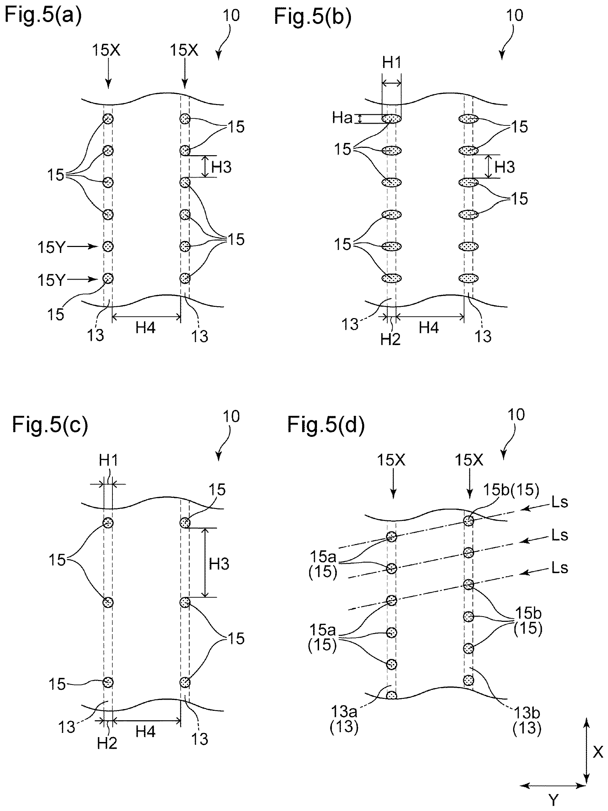

Figs. 5(a) to (d) show examples of arrangement forms of the elastic members 13 and the high-density portions 15 in the composite stretch portion 10 in the stretched state. From a viewpoint of making the aforementioned effect remarkable, as shown in Figs. 5(a) to (d), the respective high-density portions 15 preferably overlap the plurality of elastic members 13 in the thickness direction, and more preferably overlap the respective adjacent elastic members 13 in the thickness direction. In any case, the respective high-density portions 15 are also preferably formed intermittently at intervals in the extension and contraction direction X to overlap the elastic member 13.

Furthermore, the high-density portions 15 are preferably formed discontinuously at intervals in both directions of the extension and contraction direction X, and the direction to intersect the extension and contraction direction X. By having a structure like this, a formation area of the high-density portions 15 with relatively high rigidity decreases, so that it is possible to reduce marks and skin problems due to abutment of a skin of the wearer of the absorbent article and the composite stretch portion 10 by enhancing flexibility and texture of the fold structure in addition to being able to form the fold structure with high aligning performance and good appearance.

In the arrangement form shown in Fig. 5(a), the plurality of high-density portions 15 are intermittently placed along the extension and contraction direction X, and these form a first high-density portion column 15X extending along the extension and contraction direction X. A plurality of first high-density portion columns 15X are formed in the orthogonal direction Y. The thread-like elastic member 13 and the first high-density portion column 15X are placed in a position where the elastic member 13 and the high-density portion column 15X overlap each other in the thickness direction Z. Furthermore, the high-density portions 15 in Fig. 5(a) are intermittently formed along the orthogonal direction Y, and these form a second high-density portion line 15Y extending along the orthogonal direction Y.

As shown in Fig. 5(b), when the composite stretch portion 10 is seen in plan view of an XY plane in Fig. 5(b), in the high-density portion 15 overlapping the thread-like elastic member 13 in the thickness direction, a length H1 of the high-density portion 15 along the orthogonal direction Y is preferably longer than a diameter H2 that is a length of the elastic member 13 along the orthogonal direction Y. By adopting the structure like this, the sheet is prevented from rising unintentionally and the groove-shaped grooves are easily formed along the high-density portions 15, so that the fold structure with high aligning performance can be formed efficiently.

The length H1 (see Fig. 5) of the high-density portion 15 along the orthogonal direction Y is preferably 0.2 mm or more, more preferably 0.3 mm or more, and even more preferably 0.4 mm or more, and is preferably 5.0 mm or less, more preferably 3.0 mm or less, and even more preferably 2.0 mm or less. Furthermore, the diameter H2 (see Fig. 5) of the elastic member 13 is preferably 0.1 mm or more, more preferably 0.2 mm or more, and even more preferably 0.3 mm or more, and is preferably 4.0 mm or less, more preferably 3.0 mm or less, and even more preferably 2.0 mm or less.

In particular, in the high-density portion 15 that overlaps the elastic member 13 in the thickness direction, a length of the high-density portion 15 along one direction is preferably longer than a length of the high-density portion along a direction orthogonal to the one direction. In the embodiment shown in Fig. 5(b), the elliptical high-density portion 15 overlaps the elastic member 13 in the thickness direction, and is placed in a state in which a direction in which a major axis of the high-density portion 15 extends and the orthogonal direction Y correspond to each other. In the high-density portion 15 shown in Fig. 5(b), the length H1 of the high-density portion 15 along the orthogonal direction Y is longer than a length Ha of the high-density portion 15 along the extension and contraction direction X. By adopting the structure like this, the groove-shaped grooves are easily formed along the high-density portions 15, and as a result, the fold structure with high aligning performance can be formed efficiently.

From a same viewpoint, the length Ha (see Fig. 5(b)) of the high-density portion 15 along the extension and contraction direction X is preferably 0.1 mm or more, more preferably 0.2 mm or more, and even more preferably 0.3 mm or more, and is preferably 4.0 mm or less, more preferably 3.0 mm or less, and even more preferably 2.0 mm or less.

In the present invention, a relationship between the interval between the high-density portions 15 adjacent along the extension and contraction direction X and the interval between the adjacent elastic members 13 is preferably properly changed in accordance with a specific arrangement position of the composite stretch portion 10 in the absorbent article. By changing the relationship, it is possible to change the appearance and a feel of the fold structure formed by extension and contraction of the composite stretch portion 10.

In each of the forms in Figs. 5(a) and (b), an interval H3 between the high-density portions 15 adjacent along the extension and contraction direction X that is the extending direction of the elastic member 13 is smaller than an interval H4 between the elastic members 13 adjacent in the orthogonal direction Y. In other words, in each of the forms shown in Figs. 5(a) and (b), a relationship of "H3 < H4" is established. By adopting the structure like this, arrangement pitches of the ridges and grooves are formed to be short, so that the fold structure becomes fine, the aligning performance is high, and the appearance is even better. The fold structure like this is particularly useful from the viewpoint of making the appearance in the worn state good, when the composite stretch portion 10 is placed in a visually recognizable part of an outer surface or the like of the absorbent article.

Instead of this, in a form shown in Fig. 5(c), an interval H3 between the high-density portions 15 adjacent along the extension and contraction direction X that is the extending direction of the elastic member 13 is same as an interval H4 between the elastic members 13 adjacent in the orthogonal direction Y, or is larger than the interval H4. In other words, in the form shown in Fig. 5(c), a relationship "H3 = H4" or a relationship "H3 > H4" is established. By adopting the structure like this, the arrangement pitches of the ridges and the grooves are formed to be wide, so that flexibility in the ridges of the fold structure can be further enhanced, and as a result, marks and skin problems due to abutment of the skin of the wearer of the absorbent article and the composite stretch portion 10 can be further reduced. The fold structure like this is particularly useful from a viewpoint of reducing marks or the like to the skin when the composite stretch portion 10 is placed on the skin-facing surface side of the wearer.

In the case of the forms shown in Figs. 5(a) and (b), that is, when the relationship "H3 < H4" is established, the interval H3 (see Fig. 5) of the adjacent high-density portions 15 is preferably 0.5 mm or more, more preferably 1.0 mm or more, and even more preferably 1.1 mm or more, and is preferably 3.0 mm or less, more preferably 2.8 mm or less, and even more preferably 2.5 mm or less. Furthermore, the interval H4 (see Fig. 5) between the adjacent elastic members 13 is preferably 3.2 mm or more, more preferably 3.5 mm or more, and even more preferably 3.8 mm or more, and is preferably 8.0 mm or less, more preferably 7.5 mm or less, and even more preferably 7.0 mm or less.

Furthermore, in the case of the form shown in Fig. 5(c), that is, in the case of the relationship of "H3 = H4" or "H3 > H4" is established, under the condition that the interval H4 is in the aforementioned range, the interval H3 is preferably 3.8 mm or more, more preferably 4.0 mm or more and even more preferably 4.2 mm or more, and is preferably 10.0 mm or less, more preferably 9.0 mm or less, and even more preferably 8.0 mm or less.

Furthermore, in the present invention, the arrangement position or shape of the high-density portion 15 are also preferably changed, in accordance with the specific arrangement position of the composite stretch portion 10 in the absorbent article. By changing these, it is possible to improve a fit of the fold structure and the skin of the wearer, in addition to improvement of the appearance of the fold structure formed by extension and contraction of the composite stretch portion 10.

In the arrangement form shown in Fig. 5(d), as in the aforementioned respective arrangement forms, the plurality of high-density portions 15 are intermittently placed along the extension and contraction direction X in the positions overlapping the elastic members 13, and these forms a plurality of first high-density portion columns 15X extending along the extension and contraction direction X. The respective elastic members 13 and the respective first high-density portion columns 15X are placed in the positions where they overlap each other in the thickness direction Z. At this time, when focusing on the elastic members 13 and 13 adjacent in the orthogonal direction Y, arrangement positions in the extension and contraction direction X, of a plurality of first high-density portions 15a formed in positions overlapping the one elastic member 13a and a plurality of second high-density portions 15b formed in positions overlapping the other elastic members 13b are preferably deviated from each other. In the embodiment shown in Fig. 5(d), when an imaginary straight line extending along the orthogonal direction Y is drawn toward the other elastic member 13b from the one first high-density portion 15a in plan view, the second high-density portion 15b does not exist in an intersection point of the elastic member 13b and the imaginary straight line. By adopting the configuration like this, ridges and grooves that extend in a direction intersecting the extension and contraction direction X can be formed, so that fit of the fold structure and the skin of the wearer can be improved. The fold structure like this is particularly useful from a viewpoint of enhancing followability of the fold structure when the composite stretch portion 10 is placed on the skin-facing surface side of the wearer in a state of being bent to be along the body surface of the wearer.

From the viewpoint of enhancing fit of the fold structure and the skin of the wearer more, in the elastic members 13 and 13 adjacent in the orthogonal direction Y, the first high-density portions 15a overlapping the one elastic member 13a, and the second high-density portions 15b overlapping the other elastic member 13b are more preferably located on imaginary parallel lines Ls that incline with respect to both the extension and contraction direction X and the orthogonal direction Y.

In place of this, or in addition to this, both the first high-density portion 15a overlapping the one elastic member 13a, and the second high-density portion 15b overlapping the other elastic member 13b more preferably have shapes in plan view in each of which a major axis is in a direction parallel with the imaginary parallel line Ls. As the shape in plan view of the high-density portion 15 like this, the aforementioned anisotropy shapes may be cited, and from a viewpoint of forming efficiency, an oblong or an ellipse are preferable.

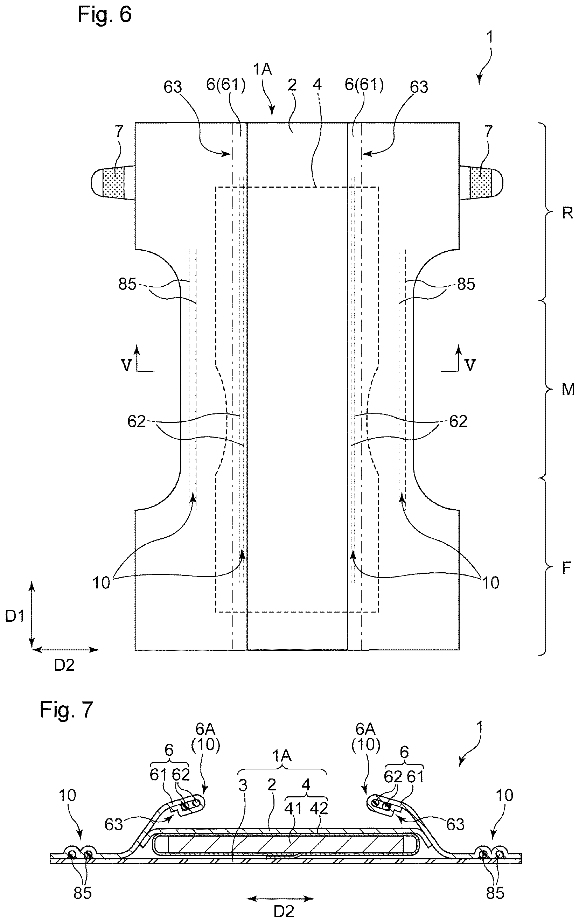

Fig. 6 and Fig. 7 show an open-type disposable diaper 1 (Hereinafter, also simply referred to as a "diaper 1."), as one embodiment of the absorbent article having the composite stretch portion 10. Fig. 6 shows a structure seen from a skin-facing surface side of the diaper 1, and the diaper 1 shown in Fig. 6 has a longitudinal direction D1 that corresponds to a front-rear direction of the wearer, and extends to a rear side from a front side of the wearer via a crotch portion, and a width direction D2 orthogonal to the longitudinal direction D1, in its flat-out, uncontracted state. The diaper 1 has a front region F, a rear region R, and a crotch region M located between them. The respective regions continuously extend in the front-rear direction of the wearer.

In the present description, the "flat-out, uncontracted state" of the diaper 1 refers to a state in which the diaper 1 is brought into a spread-open and stretched state, and the diaper 1 in its spread-out and stretched state is unfolded until the diaper 1 has design dimensions (same as dimensions when the diaper is unfolded in a plane with effects of the elastic members completely excluded) by stretching the elastic members of respective parts. Furthermore, the "skin-facing surface" is a surface that is faced to a skin of a wearer at a worn time of the diaper, when focusing on the diaper or constituent members of the diaper (for example, the absorbent member), and the "non-skin-facing surface" is a surface that is faced to an opposite side to the skin of the wearer at the worn time of the diaper. In other words, the skin-facing surface is a surface on a side relatively close to the skin of the wearer, whereas the non-skin-facing surface is a surface on a side relatively far from the skin of the wearer. The "worn time" and the "worn state" refer to a state in which the diaper is worn with a proper wear position of the diaper maintained.

As shown in Fig. 6 and Fig. 7, the diaper 1 includes a liquid-permeable topsheet 2 forming a skin-facing surface, and a sparingly liquid permeable (also including water repellent) backsheet 3 forming a non-skin-facing surface. An absorbent member 4 in a substantially oblong shape is placed between the topsheet 2 and the backsheet 3 to correspond to the longitudinal direction D1, and these form an absorbent assembly 1A. The topsheet 2 and the backsheet 3 respectively have larger dimensions than the absorbent member 4 and extend outward from an outer peripheral edge of the absorbent member 4. Furthermore, the topsheet 2 and the backsheet 3 extend outward in the width direction D2 from both side edges along the longitudinal direction D1 of the absorbent member 4. Both end edges in the longitudinal direction of the topsheet 2 and the backsheet 3 in the present embodiment substantially correspond to both end edges in the longitudinal direction of the diaper 1. The absorbent member 4 is placed from the front region F to the rear region R. An outer cover (not illustrated) forming the outer surface of the absorbent article may be further placed on the non-skin-facing surface side of the backsheet 3.

As shown in Fig. 6, a pair of leak-proof cuffs 6 and 6 are provided to extend in the longitudinal direction D1, on both sides along the longitudinal direction D1 in the skin-facing surface of the diaper 1. As shown in Fig. 6 and Fig. 7, the leak-proof cuff 6 is constituted of a leak-proof cuff forming sheet 61 that is water repellant and air permeable. At a free end portion 6A of each of the leak-proof cuffs 6, one or more thread-like cuff elastic members 62 are placed in a stretched state along the longitudinal direction D1.

As shown in Fig. 7, in the free end portion 6A in the present embodiment, the single continuous leak-proof cuff forming sheet 61 is folded to form two surface that face each other, and between the sheets 61, the cuff elastic members 62 are placed. Furthermore, a joined portion 63 that joins the leak-proof cuff forming sheets 61 is formed outward in the width direction D2 of the cuff elastic member 62. The leak-proof cuff 6 is raised in at least the crotch region M by the elastic members 62 that are placed in the stretched state contracting at the worn time of the diaper 1, and thereby inhibits excrement such as urine from flowing outward in the width direction D2.

The topsheet 2 and the backsheet 3 that are members extending outward in the width direction D2 from the absorbent member 4 form side flaps with the leak-proof cuff forming sheets 61 that are water repellent and air permeable. The side flaps are constituted of members extending outward in the width direction Y from the absorbent member 4 in the diaper 1.

As shown in Fig. 6, a pair of fastening tapes 7 and 7 are provided at both side edges along the longitudinal direction D1, of the rear region R of the diaper 1. An attachment portion formed of a male member of a mechanical hook-and-loop fastener is attached to the fastening tape 7. Furthermore, in the non-skin-facing surface of the front region F of the diaper 1, a target region (not illustrated) formed of a female member of the mechanical hook-and-loop fastener is formed. The target region is formed by joining and fixing the female member of the mechanical hook-and-loop fastener to the backsheet 3 forming the non-skin-facing surface of the front region F or the non-skin-facing surface of the outer cover by known joining means, for example, an adhesive, a heat seal or the like. The attachment portion of the fastening tape 7 is detachably engageable with the target region.

As shown in Fig. 6, in each of both sides along the longitudinal direction D1 of the diaper 1, a plurality of leg elastic members 85 for forming leg gathers are placed in a stretched state along the longitudinal direction D1, between the two sheets constituting the diaper 1, so that leg gathers can be formed by contraction of the leg elastic members 85. In the present embodiment, no elastic member exists in the respective longitudinal end portion regions in the front region F and the rear region R of the diaper 1, but, for example, a plurality of below-waist elastic members may be placed in the stretched state in the width direction D2 between the two sheets constituting the longitudinal end regions of the diaper 1 so that waist gathers can be formed by contraction of the elastic members.

In the diaper 1 having the form like this, the aforementioned composite stretch portion 10 is formed in the parts in which the sheets and the elastic members are placed such as the leak-proof cuffs 6, the leg gathers, and the waist gathers.

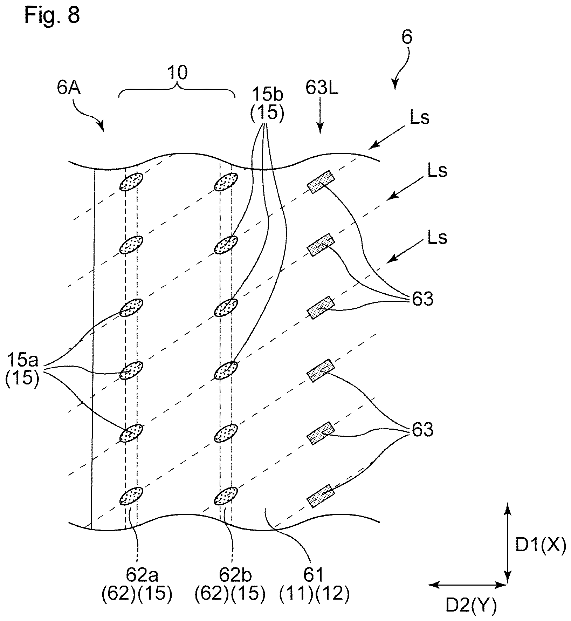

Hereinafter, the diaper 1 in which the composite stretch portion 10 is placed in the leak-proof cuffs 6 is taken as an example, and is described with reference to Fig. 8. Fig. 8 shows an essential part enlarged view of one of the leak-proof cuffs 6 in a flat-out, uncontracted state of the diaper 1 seen from a skin-facing surface side. The leak-proof cuff 6 has the composite stretch portion 10 in the free end portion 6A thereof, and is placed so that the extension and contraction direction X of the composite stretch portion 10 and the longitudinal direction D1 of the diaper 1 correspond to each other, and the orthogonal direction Y of the composite stretch portion 10 and the width direction D2 of the diaper 1 correspond to each other. Furthermore, a plurality of joined portions 63 that join the leak-proof cuff forming sheets 61 in a folded state are intermittently formed in the longitudinal direction D1 to form a joined portion column 63L extending along the longitudinal direction D1. A shape in plan view of the joined portion 63 may be a rectangular shape as shown in Fig. 8, may be a true circle or an ellipse, or may be a polygonal shape such as a triangle and a pentagon.

A plurality of high-density portions 15 are formed in the leak-proof cuff forming sheet 61, and these high-density portions 15 are placed to overlap a plurality of cuff elastic members 62 in a thickness direction. At this time, first high-density portions 15a that overlap one cuff elastic member 62a adjacent in the width direction D2 in the thickness direction, a plurality of second high-density portions 15b that overlap the other cuff elastic member 62b, and the respective joined portions 63 are preferably located on imaginary parallel lines Ls that incline to both the longitudinal direction D1 and the width direction D2. Thereby, fit of the leak-proof cuff 6 in which the fold structure is formed and the skin of the wearer can be more enhanced.

In particular, as shown in Fig. 6, the plurality of high-density portions 15 each preferably has a shape in plan view in which a long side or a major axis is in a direction parallel with the imaginary parallel lines Ls, and in addition to this, the respective joined portions 63 each more preferably has a shape in plan view in which a long side or a major axis is in a direction parallel with the imaginary parallel line Ls. In the embodiment shown in Fig. 8, the respective high-density portions 15 each has an elliptical shape in plan view, and the major axis of the ellipse and the imaginary line Ls are parallel with each other. Likewise, the respective joined portions 63 each has a rectangular shape in plan view, and a long side of the rectangle and the imaginary parallel line Ls are parallel with each other. Instead of this, for example, both of each of the respective high-density portions 15 and each of the respective joined portions 63 may be in a rectangular or elliptical shape in plan view, and one of the high-density portion 15 and the joined portion 63 may be in a rectangular shape in plan view, and the other one may be in an elliptical shape in plan view.

While the leak-proof cuff 6 that is placed in an absorbent article such as the diaper 1 is desired to be formed so that a space is not made between the leak-proof cuff 6 and the skin of the wearer in order to prevent excrement such as urine from flowing outward in the width direction D2, it is possible to form the fold structure in which phases of the high-density portions and the joined portions correspond to each other, aligning performance is high, and the appearance is more excellent by using the composite stretch portion 10 having the aforementioned arrangement as the leak-proof cuff 6. In addition to this, the fold structure has a ridge groove shape along the direction in which the imaginary parallel line Ls extends, so that even in a state where the absorbent article bends, the fold structure easily follows the wearer, and fit of the leak-proof cuffs in which the fold structure is formed and the skin of the wearer can be more enhanced.

Hereinafter, matters applicable to the respective embodiments in common will be described.

As for the sheets constituting the composite stretch portion 10, ring crush compressive strength that is measured by a method described later is preferably 10.0 N/30 mm or less, more preferably 8.0 N/30 mm or less, even more preferably 7.5 N/30 mm or less, and although the lower the lower limit thereof, the better, 2.0 N/30 mm or more is realistic. By having the properties like this, rigidity of the sheets themselves constituting the composite stretch portion 10 is reduced, flexibility and texture of the fold structure to be formed are enhanced, and marks due to abutment of the skin of the wearer of the absorbent article and the composite stretch portion 10 can be reduced.

Here, the ring crush compressive strength can be measured as follows. First, five pieces were cut out from the sheet constituting the composite stretch portion 10 into an oblong shape with a length of 150 mm in the extension and contraction direction X, and a length of 30 mm in the orthogonal direction Y, and these were used as test pieces. Each of the test pieces was rolled into a cylindrical shape in a longitudinal direction of the test piece, one end and the other end in the longitudinal direction of the test piece were overlapped by 5 mm to make a cylinder with a diameter of 45 mm, two spots of an upper end (an upper end in a cylinder axis direction) and a lower end of overlapped portions of both the ends are fastened by using staples [for example, staples of No. 10-1M made by MAX Co., Ltd.] to obtain five cylindrical measurement samples. Note that a direction of the staples is parallel with a circumferential direction. Each of the obtained measurement samples (initial sample width; 30 mm) was set in a Tencilon compression tester ("RTA-100" made by ORIENTEC CO., LTD.), and a maximum load (N/30 mm) that is shown when compressed at a compression speed of 10 mm/sec. in the axial direction (width direction of the sample) of the cylinder by a circular compression plate with a diameter of 70 mm or more was measured. Similar measurement is performed with respect to the five measurement samples, and an arithmetic mean value of them is determined as the ring crush compressive strength.

A basis weight of the entire composite stretch portion 10 is preferably 10 g/m² or more, more preferably 12 g/m² or more, and even more preferably 15 g/m² or more, and is preferably 40 g/m² or less, more preferably 36 g/m² or less, and even more preferably 32 g/m² or less, from a viewpoint of improving flexibility and texture.

As the sheets 11 and 12 constituting the composite stretch portion 10, various fiber sheets including a nonwoven fabric can be used, for example. As the nonwoven fabric, it is possible to use known nonwoven fabrics such as a spun bond nonwoven fabric, a thermal bond nonwoven fabric (a point bond nonwoven fabric, a heat embossed nonwoven fabric, an air-through nonwoven fabric and the like), a spun lace nonwoven fabric, a melt blown nonwoven fabric, an air-laid nonwoven fabric, a resin bond nonwoven fabric, a needle punch nonwoven fabric, and a wet nonwoven fabric without particular limitation, for example. The sheets may be a single layer or a multi-layer. Further, a laminate of the aforementioned fiber sheet and a film may be used.

When the respective sheets 11 and 12 are nonwoven fabrics, fibers constituting the nonwoven fabrics can be constituted of various thermoplastic resins, for example, a polyolefin resin such as polyethylene, polypropylene, and polybutene, a polyester resin such as polyethylene terephthalate, and polybutylene terephthalate, a polyamide resin such as nylon, a vinyl resin such as polystyrene and polyvinyl chloride, an acrylonitrile resin such as acryl, a methacrylic resin, a vinylidene resin, a natural fiber cotton, pulp, biodegradable plastics such as polylactic acid, and the like. Alternatively, it is possible to constitute fibers from a blend of two or more kinds of these resins, or use composite fibers obtained by combining two or more kinds of these resins (core sheath type fiber, side-by-side type fiber, core sheath fiber having an eccentric crimp, and split fiber). Furthermore, it is possible to use fibers obtained by adding a small amount of additive such as a fiber coloring agent, an antistatic agent, a lubricant, a slip additive, or a hydrophilic agent to fibers.

The elastic member 13 constituting the composite stretch portion 10 can be constituted of an elastic resin, for example. As the elastic resin, there may be cited, for example, a synthetic rubber such as a styrene-butadiene rubber, butadiene rubber, isoprene rubber and neoprene rubber, a natural rubber, EVA rubber, SIS (styreneisoprene-styrene) rubber, SEBS (styrene-ethylene-butylene-styrene) rubber, SEPS (styrene-ethylene-propylene-styrene) rubber, elastic polyolefin (the ethylene elastomer, the propylene elastomer and the like), polyurethan and the like. As a shape, a sheet shape such as a film shape, and a net shape, a thread shape or a string shape (flat rubber) with a rectangular, square, circular or polygonal section, a multifilament type of thread shape or the like can be used.

The number of elastic members placed in the composite stretch portion 10 is preferably two or more and more preferably three or more, and is preferably 20 or less, and more preferably 10 or less. As for a way of counting the number of elastic members, when an imaginary straight line is drawn in a direction orthogonal to the extending direction of the elastic members, the number of elastic members that intersect the imaginary straight line is defined as the number of elastic members described above. When the composite stretch portions 10 exist in a plurality of spots in the absorbent article, a range of the aforementioned number can be independently satisfied in each of the spots.

In the composite stretch portion 10 in the contracted state, a height W1 (see Fig. 4) of the first ridge 17a is preferably 0.2 mm or more and more preferably 0.5 mm or more, and is preferably 5.0 mm or less and more preferably 3.0 mm or less. A height H1 of a ridge 51 is a distance between a surface that forms a groove 52 in an outer sheet 5, and a top surface of a top portion of the ridge 51. A height of the second ridge 17b can be also within a same range as the height W1. The height W1 of each of the ridges 17a and 17b can be properly adjusted by changing the interval H3 between the high-density portions 15 that are adjacent along the extension and contraction direction X, for example.

Furthermore, from the same viewpoint, an entire width W2 (see Fig. 4) along the extension and contraction direction X of the first ridge 17a is preferably 0.5 mm or more, and more preferably 1.0 mm or more, and is preferably 3.0 mm or less and more preferably 2.5 mm or less. An entire width W3 (see Fig. 4) along the extension and contraction direction X of the first groove 18a is preferably 0.2 mm or more and more preferably 0.4 mm or more, and is preferably 5.0 mm or less and more preferably 3.0 mm or less. The entire widths W1 and W2 can be properly adjusted by changing the interval H3 between the high-density portions 15 adjacent along the extension and contraction direction X, and the size of the shape in plan view of the high-density portion 15. When the second ridge 17b and the second groove 18b are formed, these can have same widths in the ranges of the entire widths W2 and W3.

The height W1, the entire width W2 and the entire width W3 described above can be measured by the following method, for example. In other words, a sample having the composite stretch portion 10 in the contracted state is cut along the extension and contraction direction X with a sharp razor, a cut surface thereof is observed, a shortest distance between a surface forming the groove and a top surface of a top portion of the ridge is measured, and an obtained value is determined as the height W1. Furthermore, in the cut surface, a distance between the high-density portions 15 and 15 adjacent in a direction orthogonal to the extension and contraction direction X is measured, and this is determined as the entire width W2. Furthermore, in the cut surface, a shortest length of the groove in the direction orthogonal to the extension and contraction direction X is measured, and this is determined as the entire width W3. When measurement is difficult with naked eyes, a section of the cut sample may be observed and measure by power of 20 to 100 by using a microscope (VHX-1000 made by KEYENCE CORPORATION), for example.

In the composite stretch portion 10, the number of elastic members 13 and first high-density portion columns 15X overlapping each other is preferably one or more, more preferably two or more, and even more preferably three or more, and is preferably 20 or less, more preferably 15 or less, and even more preferably 10 or less. Thereby, it is possible to form the fold structure with high aligning performance and good appearance.

As one embodiment of the absorbent article of the present invention, the composite stretch portion 10 is descried as being formed in an open-type disposable diaper, but the arrangement position of the composite stretch portion 10 and the kind of the absorbent article to which the composite stretch portion 10 is applied are not particularly limited. For example, the absorbent article may be a pull-on disposable diaper, and the composite stretch portion 10 may be formed in the parts such as leg gathers, waist gathers and the leak-proof cuffs. Furthermore, the composite stretch portion 10 may be formed in the parts such as leak-proof cuffs in an incontinence pad.

The present invention is described above based on preferable embodiments thereof, but the present invention is not limited by the aforementioned embodiments. For example, as another embodiment of the open-type disposable diaper 1, there may be cited a mode in which a pair of elastic side panels extending outward in the width direction D2 are provided at both sides along the longitudinal direction D1 of the rear region R of the absorbent assembly 1A, and a fastening tape extending outward in the width direction D2 is attached to an outward side portion in the width direction D2 of each of the side panels. In this mode, the composite stretch portions 10 can be used in parts where the elastic side panels are placed.

Concerning the embodiments of the present invention described above, an absorbent article as follows is further disclosed.
<1> An absorbent article comprising a liquid-permeable topsheet, a backsheet and an absorbent member located between the topsheet and the backsheet, and including a longitudinal direction corresponding to a front-rear direction of a wearer and a width direction orthogonal to the longitudinal direction,
   the absorbent article comprising a composite stretch portion capable of extending and contracting in one direction,
   wherein the composite stretch portion includes a first sheet, a second sheet, and a plurality of elastic members placed between the first sheet and the second sheet,
   the plurality of elastic members extend along the one direction, and are placed at intervals in a direction orthogonal to the one direction,
   at least one sheet of the first sheet and the second sheet includes a plurality of high-density portions that do not directly join the first sheet and the second sheet to each other,
   the high-density portions overlap at least one elastic member of the plurality of elastic members, and the plurality of high-density portions are intermittently formed in an extending direction of the one elastic member, and
   in the composite stretch portion in a contracted state, a fold structure in which the one sheet is deformed so as to have a plurality of grooves each including the high-density portion in a bottom portion and ridges located between the grooves is formed.
<2> The absorbent article as set forth in clause <1>, wherein the high-density portions are formed by densification processing.
<3> The absorbent article as set forth in clause <1> or <2>, wherein the high-density portions overlap the plurality of elastic members, and are intermittently formed in the one direction in which each of the plurality of elastic members extends.
<4> The absorbent article as set forth in clause <3>, wherein the high-density portions overlap the plurality of adjacent elastic members, and are intermittently formed in the one direction in which each of the plurality of elastic members extends.
<5> The absorbent article as set forth in any one of clauses <1> to <4>, wherein the high-density portions are discontinuously formed in both directions of the one direction and a direction that intersects the one direction.
<6> The absorbent article as set forth in any one of clauses <1> to <5>,
   wherein the elastic members are thread-like members, and
   in a shape in plan view of the composite stretch portion, a length in the direction orthogonal to the one direction, of the high-density portion is longer than a diameter of the elastic member.
<7> The absorbent article as set forth in any one of clauses <1> to <5>, wherein an interval between the high-density portions that are adjacent along the extending direction of the elastic member is smaller than an interval between the adjacent elastic members.
<8> The absorbent article as set forth in clause <7>, wherein the interval between the adjacent high-density portions is 0.5 mm or more and 3.0 mm or less, preferably 1.0 mm or more and 2.8 mm or less, and more preferably 1.1 mm or more and 2.5 mm or less.
<9> The absorbent article as set forth in clause <7> or <8>, wherein the interval between the adjacent elastic members is 3.2 mm or more and 8.0 mm or less, preferably 3.5 mm or more and 7.0 mm or less, and more preferably 3.8 mm or more and 7.0 mm or less.
<10> The absorbent article as set forth in any one of clauses <1> to <6>, wherein an interval between the high-density portions that are adjacent along the extending direction of the elastic member is same as an interval between the adjacent elastic members, or larger than the interval between the adjacent elastic members.
<11> The absorbent article as set forth in clause <10>, wherein the interval between the adjacent high-density portions is 3.8 mm or more and 10.0 mm or less, preferably 4.0 mm or more and 9.0 mm or less, and more preferably 1.1 mm or more and 2.5 mm or less.
<12> The absorbent article as set forth in clause <10> or <11>, wherein the interval between the adjacent elastic members is 3.2 mm or more and 8.0 mm or less, preferably 3.5 mm or more and 7.0 mm or less, and more preferably 4.2 mm or more and 8.0 mm or less.
<13> The absorbent article as set forth in any one of clauses <1> to <12>,
   wherein the high-density portions overlap the plurality of elastic members, and
   arrangement positions in the one direction of a plurality of high-density portions overlapping one of elastic members adjacent in the direction orthogonal to the one direction, and a plurality of high-density portions overlapping the other elastic member deviate from each other.
<14> The absorbent article as set forth in clause <13>, wherein the high-density portion overlapping the one elastic member and the high-density portion overlapping the other elastic member are located on an imaginary parallel line inclining to both directions of the one direction and the direction orthogonal to the one direction.
<15> The absorbent article as set forth in clause <14>, wherein both the high-density portion overlapping the one elastic member and the high-density portion overlapping the other elastic member have a shape in plan view in each of which a major axis is in a direction parallel with the imaginary parallel line.
<16> The absorbent article as set forth in any one of clauses <1> to <15>,
   wherein the absorbent article comprises a leak-proof cuff extending in the longitudinal direction,
   the leak-proof cuff includes the composite stretch portion in a free end portion thereof, and the composite stretch portion is placed so that the one direction in which the plurality of elastic members extend extends in the longitudinal direction of the absorbent article,
   joined portions that join the first sheet and the second sheet are intermittently formed in the longitudinal direction, outward from the plurality of elastic members,
   the high-density portions overlap the plurality of elastic members, and
   the plurality of high-density portions that overlap one of the elastic members adjacent in the direction orthogonal to the one direction, the plurality of high-density portions that overlap the other elastic member, and the joined portions are located on imaginary parallel lines inclining to both directions of the one direction and the direction orthogonal to the one direction.
<17> The absorbent article as set forth in any one of clauses <1> to <16>, wherein ring crush compressive strength of each of the first sheet and the second sheet is 10.0 N/30 mm or less, preferably 8.0 N/30 mm or less, and more preferably 7.5 N/30 mm or less.
<18> The absorbent article as set forth in any one of clauses <1> to <17>, wherein in the high-density portion that overlaps the elastic member in a thickness direction, a length of the high-density portion along the one direction is longer than a length of the high-density portion along the direction orthogonal to the one direction.
<19> The absorbent article as set forth in clause <18>, wherein the length of the high-density portion along the one direction is 0.2 mm or more and 5.0 mm or less, preferably 0.3 mm or more and 3.0 mm or less, and more preferably 0.4 mm or more and 2.0 mm or less.
<20> The absorbent article as set forth in clause <18> or <19>, wherein the length of the high-density portion along the direction orthogonal to the one direction is 0.1 mm or more and 4.0 mm or less, preferably 0.2 mm or more and 3.0 mm or less, and more preferably 0.3 mm or more and 2.0 mm or less.

### Industrial Applicability

According to the present invention, the absorbent article in which the fold structure with high aligning performance and excellent appearance is formed is provided.

## Claims

1. An absorbent article comprising a liquid-permeable topsheet, a backsheet and an absorbent member located between the topsheet and the backsheet, and including a longitudinal direction corresponding to a front-rear direction of a wearer and a width direction orthogonal to the longitudinal direction,
the absorbent article comprising a composite stretch portion capable of extending and contracting in one direction,
wherein the composite stretch portion includes a first sheet, a second sheet, and a plurality of elastic members placed between the first sheet and the second sheet,
the plurality of elastic members extend along the one direction, and are placed at intervals in a direction orthogonal to the one direction,
at least one sheet of the first sheet and the second sheet includes a plurality of high-density portions that do not directly join the first sheet and the second sheet to each other,
the high-density portions overlap at least one elastic member of the plurality of elastic members, and the plurality of high-density portions are intermittently formed in an extending direction of the one elastic member, and
in the composite stretch portion in a contracted state, a fold structure in which the one sheet is deformed so as to have a plurality of grooves each including the high-density portion in a bottom portion and ridges located between the grooves is formed.

2. The absorbent article according to claim 1, wherein the high-density portions are formed by densification processing.

3. The absorbent article according to claim 1 or 2, wherein the high-density portions overlap the plurality of elastic members, and are intermittently formed in the one direction in which each of the plurality of elastic members extends.

4. The absorbent article according to claim 3, wherein the high-density portions overlap the plurality of adjacent elastic members, and are intermittently formed in the one direction in which each of the plurality of elastic members extends.

5. The absorbent article according to any one of claims 1 to 4, wherein the high-density portions are discontinuously formed in both directions of the one direction and a direction that intersects the one direction.

6. The absorbent article according to any one of claims 1 to 5,
wherein the elastic members are thread-like members, and
in a shape in plan view of the composite stretch portion, a length in the direction orthogonal to the one direction, of the high-density portion is longer than a diameter of the elastic member.

7. The absorbent article according to any one of claims 1 to 5, wherein an interval between the high-density portions that are adjacent along the extending direction of the elastic member is smaller than an interval between the adjacent elastic members.

8. The absorbent article according to claim 7, wherein the interval between the adjacent high-density portions is 0.5 mm or more and 3.0 mm or less.

9. The absorbent article according to claim 7 or 8, wherein the interval between the adjacent elastic members is 3.2 mm or more and 8.0 mm or less.

10. The absorbent article according to any one of claims 1 to 6, wherein an interval between the high-density portions that are adjacent along the extending direction of the elastic member is same as an interval between the adjacent elastic members, or larger than the interval between the adjacent elastic members.

11. The absorbent article according to claim 10, wherein the interval between the adjacent high-density portions is 3.8 mm or more and 10.0 mm or less.

12. The absorbent article according to claim 10 or 11, wherein the interval between the adjacent elastic members is 3.2 mm or more and 8.0 mm or less.

13. The absorbent article according to any one of claims 1 to 12,
wherein the high-density portions overlap the plurality of elastic members, and
arrangement positions in the one direction of a plurality of high-density portions overlapping one of elastic members adjacent in the direction orthogonal to the one direction, and a plurality of high-density portions overlapping the other elastic member deviate from each other.

14. The absorbent article according to claim 13, wherein the high-density portion overlapping the one elastic member and the high-density portion overlapping the other elastic member are located on an imaginary parallel line inclining to both directions of the one direction and the direction orthogonal to the one direction.

15. The absorbent article according to claim 14, wherein both the high-density portion overlapping the one elastic member and the high-density portion overlapping the other elastic member have a shape in plan view in each of which a major axis is in a direction parallel with the imaginary parallel line.

16. The absorbent article according to any one of claims 1 to 15,
wherein the absorbent article comprises a leak-proof cuff extending in the longitudinal direction,
the leak-proof cuff includes the composite stretch portion in a free end portion thereof, and the composite stretch portion is placed so that the one direction in which the plurality of elastic members extend extends in the longitudinal direction of the absorbent article,
joined portions that join the first sheet and the second sheet are intermittently formed in the longitudinal direction, outward from the plurality of elastic members,
the high-density portions overlap the plurality of elastic members, and
the plurality of high-density portions that overlap one of the elastic members adjacent in the direction orthogonal to the one direction, the plurality of high-density portions that overlap the other elastic member, and the joined portions are located on imaginary parallel lines inclining to both directions of the one direction and the direction orthogonal to the one direction.

17. The absorbent article according to any one of claims 1 to 16, wherein ring crush compressive strength of each of the first sheet and the second sheet is 10.0 N/30 mm or less.

18. The absorbent article according to any one of claims 1 to 17, wherein in the high-density portion that overlaps the elastic member in a thickness direction, a length of the high-density portion along the one direction is longer than a length of the high-density portion along the direction orthogonal to the one direction.

19. The absorbent article according to claim 18, wherein the length of the high-density portion along the one direction is 0.2 mm or more and 5.0 mm or less.

20. The absorbent article according to claim 18 or 19, wherein the length of the high-density portion along the direction orthogonal to the one direction is 0.1 mm or more and 4.0 mm or less.
